# EUROPEAN PATENT APPLICATION

(11) **EP 2 055 256 A1**
(43) Date of publication of application: **06.05.2009**
(21) Application number: 08164947.7
(22) Date of filing: 23.09.2008
(51) Int. Cl.: A61B 19/02, A61B 17/70

(54) **A packaging system for shape memory alloy components**

(30) Priority: 02.11.2007 GB 0721539
(71) Applicant: DePuy Spine Sàrl, 2400 Le Locle (CH)
(72) Inventor: Defossez, Henri, CH-2400 Le Locle (CH); Sanders, Marc, CH-2400 Le Locle (CH)
(74) Representative: Belcher, Simon James

(57) **Abstract**

A packaging system for shape memory alloy components for use in a surgical procedure, includes a case (2,102) comprising a base part (4) and a lid part (6) and a seal (14) to prevent ingress of contaminants when the base and lid parts are closed, a heat sink which can be fitted snugly in the case and which is formed from a metal, and at least one component which is formed from a shape memory alloy, which can be fitted in the case in heat exchange relationship with the heat sink.

## Description

This invention relates to a packaging system for shape memory alloy components for use in a surgical procedure.

Support can be used to correct serious shape deformations of the spinal column and chest of a patient. Examples of serious shape deformations of the spinal column include lateral curvature and axial turning.

EP-A-470660 discloses apparatus for correcting the shape of a spinal column. The apparatus includes a rod having a square cross-section. The rod is formed from a nickel-titanium alloy which has been treated so that it exhibits shape memory properties, in bending or in torsion or both. Articles formed from shape memory alloys can exhibit shape memory properties associated with transformations between martensite and austenite phases of the alloys. These properties include thermally induced changes in configuration in which an article is first deformed from a heat-stable configuration to a heat-unstable configuration while the alloy is in its martensite phase. Subsequent exposure to increased temperature results in a change in configuration from the heat-unstable configuration towards the original heat-stable configuration as the alloy reverts from its martensite phase to its austenite phase. The transformation from austenite to martensite on cooling begins at a temperature known as the Mₛ temperature, and is completed at a temperature known as the M_{f} temperature. The transformation of martensite to austenite upon heating begins at a temperature known as the Aₛ temperature and is complete at a temperature known as the A_{f} temperature.

The rod of the apparatus disclosed in EP-A-470660 is fastened to a patient's vertebrae while in the configuration from which it has to recover. The rod can be bent and twisted so as to correspond to the curve or torsion of the deformed spinal column. The temperature of the rod is then increased so that it is greater than the Aₛ temperature of the alloy. The rod then recovers towards its heat-stable configuration, applying a corrective force to the spinal column.

It is important to be able to control the temperature of shape memory alloy rods prior to implantation in a patient, for example to be able to deform the rods prior to implantation when in the martensite phase to match the shape of the patient's spine.

The present invention provides a packing solution which includes a heat sink which can be used to maintain the temperature of shape memory alloy rods below the Aₛ temperature of the alloy prior to implantation.

Accordingly, in one aspect, the invention provides a packaging system for shape memory alloy components for use in a surgical procedure, which comprises:
a. a case comprising a base part and a lid part and a seal to prevent ingress of contaminants when the base and lid parts are closed,
b. a heat sink which can be fitted snugly in the case and which is formed from a metal, and
c. at least one component which is formed from a shape memory alloy, which can be fitted in the case in heat exchange relationship with the heat sink.

The system of the invention has the advantage that a shape memory alloy component can be maintained at a temperature which is less than the Aₛ temperature of the alloy prior to implantation. The system can also enable the temperature of a component which has been allowed to rise above the Aₛ (or A_{f}) temperature of the alloy to be reduced to a level which is below that temperature, or below the M_{f} (or Mₛ) temperature of the alloy, or below each of the Aₛ and M_{f} temperatures of the alloy. This is facilitated by the relatively high specific heat capacities which are a feature of many metals. Particularly preferred metals for use in the heat sink of the system include aluminium and certain alloys thereof, certain stainless steels, and titanium and its alloys.

Preferably, the metal of the heat sink has a specific heat capacity of at least about 400 J.kg⁻¹.K⁻¹, more preferably at least about 750 J.kg⁻¹.K⁻¹, especially at least about 900 J.kg⁻¹.K⁻¹.

Preferably, the heat capacity of the heat sink is at least about 700 J.kg⁻¹, more preferably at least about 800 J.kg⁻¹, especially at least about 900 J.kg⁻¹, for example at least about 1000 J.kg⁻¹.

Preferably, the metal of the heat sink has a thermal conductivity of at least about 15 W.m⁻¹.K⁻¹, more preferably at least about 95 W.m⁻¹.K⁻¹, especially at least about 150 W.m⁻¹.K⁻¹, for example at least about 225 W.m⁻¹.K⁻¹.

Preferably, the metal of the heat sink has a density of not more than about 3.5 g.cm⁻³, more preferably not more than about 3.0 g.cm⁻³, especially not more than about 2.8 g.cm⁻³.

The heat sink should be a snug fit in the case so that most of the volume of the case is occupied by the heat sink, apart from space within the case which might be occupied by the shape memory alloy component and possibly other components such as separator components as discussed in more detail below. For example, it can be preferred that the ratio of the volume of the case to the volume which is occupied by the material of the heat sink is at least about 0.6, more preferably at least about 0.7, especially at least about 0.8. Generally, the shape of the heat sink will correspond generally to the shape which is defined internally by the case so that the clearance between the external surface of the heat sink and the internal wall of the case is sufficient to allow the heat sink to be slid into the case, and to be a sliding fit therein.

The system of the invention can be used to prepare spinal support rods for implantation in a patient. It can be used to prepare other shape memory alloy components for use in surgery, for example components which are used in orthopaedic applications (such as bone plates and bone anchors), or in dental applications (such as dental arch wires), or in cardiovascular applications (such as stents).

Preferably, the heat sink has a recess formed in it, and in which the shape memory alloy component can be fitted in the recess. Preferably, the component should be a snug fit in the recess in the heat sink over at least part of the surface area of the component, so that the shape of the recess matches approximately the shape of the component. For example, when the component is a rod, the recess can be in the form of a groove. Each of the rod and the groove will frequently have approximately a constant cross-section along most of its length, and the ratio of the cross-sectional area of the rod to the ratio of the cross-sectional area of the groove might then be at least about 0.6, preferably at least about 0.7, especially at least about 0.8. For example, when the rod has a square cross-section with a width of 6.33 mm, the groove might have a width of 7.00 mm. A local portion of the rod might have a larger cross-section, for example to provide a socket in which an end of a rod can be received. A portion of the groove might have a larger cross-section, for example to enable a user to grip a rod which is located in the groove, or to accommodate a portion of the rod which has a larger cross-section.

Preferably, the ratio of the volume of the recess in the heat sink to the volume of the heat sink is not more than about 0.3, more preferably not more than about 0.2, for example not more than about 0.1.

The rod (or one or more of the rods when the system includes more than one rod) can be straight. The rod (or one or more of the rods when the system includes more than one rod) can be curved.

The length of the rod is preferably at least about 30 mm, more preferably at least about 50 mm, for example at least about 80 mm, and possibly 120 mm or more. The length of the rod might be not more than about 180 mm, preferably not more than about 150 mm.

The cross-sectional area of rod will often be approximately constant over at least most of the length of the rod , with the possibility that the cross-section might vary in at least one end region to facilitate connection directly or indirectly to a vertebra at the end or to an adjacent support element. The cross-sectional area of the rod might be at least about 30 mm², preferably at least about 50 mm², more preferably at least about 70 mm², for example about 100 mm².

The cross-sectional shape of the rod might be generally rounded, especially circular. It can be preferred for a rod sometimes to be non-circular to enable it to fit securely in a fastener in such a way that it can transmit torque to the fastener. For example, the element can have at least one flat face. Polygonal (regular or irregular) shapes can be useful, for example with at least four faces, including square or rectangular or trapezoidal (when the element has four faces when viewed in cross-section), or with six or eight or more faces. An rod which has a generally rounded cross-section might have a flat face.

An example of a preferred rod has a square cross-section, in which the width of the rod is 6.33 mm². The length of useful rods might be for example 300 mm and 150 mm.

Preferably, the heat sink has at least two grooves formed in it, and the packaging system includes two rods which are formed from a shape memory alloy, which can be fitted in respective ones of the grooves in the heat sink. The rods can differ from one another in one or more of their lengths, curvatures, cross-section shapes, and their cross-section areas.

At least one of the base part and the lid part of the case can be formed from a metal. Preferably, both of the base part and the lid part are formed from metallic materials, more preferably the same material, so that the lid and base parts have the same thermal expansion characteristics. Use of a metal can have the advantage of facilitating cleaning of the case without causing significant deterioration of the material of the case. Examples of suitable materials for the case include aluminium and certain alloys thereof, certain stainless steels, and titanium and its alloys. The base part or the lid part of the case might be made from a polymeric material. Suitable polymeric materials should be capable of withstanding mistreatment during preparation for use and subsequent use, including cleaning and sterilisation treatments. Examples of suitable polymeric materials include certain polyolefins (especially polyethylenes and polypropylenes), polyesters, polyamides, polycarbonates and so on.

The seal between the lid and base parts of the case can be provided by a compressible gasket. The gasket can be located in a groove which is provided in one or more of the lid part and the base part. The gasket can be provided by an elastomeric material such as a rubber, especially a silicone rubber. Preferably, the cross-sectional area of the gasket is less than the cross-sectional area of the groove, between the lid and the base parts of the case.

The case should preferably include a locking arrangement to retain the lid and base parts closed. The case can have one or more hinges along one edge and one more locking devices along one or more other edges. The case can have one or more locking devices along each of two opposite edges. Locking devices for retaining lid and base parts of trays for surgical instruments are known and can be suitable for use with the case of the present system, subject to appropriate selection as to size, materials, configuration and so on.

The base part or the lid part of the case, or each of them, will often define a recess in which a respective part of the heat sink can be received. Preferably, the depth of the recess is approximately equal to the depth of the part of the heat sink which is to fit into that part of the case so that the heat sink is approximately flush with the edge of the case (except that it can be preferred to provide a space to accommodate a layer of separator material between the parts of the heat sink in some applications).

Preferably, the system includes a separator provided by a layer of an insulating material which can be positioned between the heat sink and the case. Preferably, a layer of separator is provided for positioning between the lid part of the heat sink and the lid part of the case. Preferably, a layer of separator is provided for positioning between the base part of the heat sink and the base part of the case. The use of a layer of an insulating material can help to insulate a user of the system from the cold temperature of the heat sink. It can also help to reduce the likelihood of components of the system sticking to one another such as would prevent separation.

Preferably, the system includes a separator provided by a material which is resiliently compressible. Preferably, a layer of separator is provided for positioning between the lid part of the heat sink and the lid part of the case. Preferably, a layer of separator is provided for positioning between the base part of the heat sink and the base part of the case. The use of a material which is resiliently compressible can help to ensure that the heat sink is held securely without unwanted movement within the case. The separator can be in the form of a foam material, or in the form of a sheet with a plurality of resiliently deformable studs arranged on the sheet in a two dimensional array. A preferred material for the separator comprises a silicone rubber. The use of certain elastomeric materials, especially a silicone rubber, has the advantage that a low coefficient of expansion which means that the fit of the heat sink with the or each separator in the case will not be affected adversely to a significant degree due to changes in the temperature of the heat sink or the case or each of them.

Preferably, a quantity of separator is provided for positioning between lid and base parts of the heat sink. The separator which is provided between the lid and base parts of the heat sink can be provided as an extended layer of material which extends over at least about 50%, preferably at least about 75%, for example at least about 90% of the area of the interface between the lid and base parts of the heat sink. The separator can be provided locally in the form of localised spacers which maintain a small gap between the lid and base parts. Such spacers might be made from a material which is resistant to significant compression so as to maintain that small gap.

The rod can be used in a spinal support system which includes components for fastening the rod to a patient's vertebrae, for example in the form of hooks or screws. Such fastening components and other components of a spinal support system are known.

Preferably, one or each of the parts of the heat sink has formations which enable it to be gripped when the system is in use, especially to place the heat sink part in a respective part of the case, or to remove it from within the part of the case. For example, a stud can be provided in a recess in the heat sink part, which can be gripped using an appropriate socket in which the stud can be received. For example, the stud can be provided in the form of an AO connector.

Accordingly, it can be preferred that the heat sink has an instrument recess formed in it, and that the packaging system includes an instrument which can be positioned in the recess to engage the heat sink, so that the heat sink can be manipulated. The recess can contain a stud which can be gripped using the instrument to manipulate the heat sink, for example to assemble the system of the invention after the heat sink has been cooled, or to open the system of the invention to retrieve a cooled component from within the recess in the heat sink.

The case and heat sink can have a generally rounded cross-section, for example a generally circular cross-section. Then the case and heat sink have a rounded cross-section, especially a circular cross-section, the lid and base parts can have a half-round cross-section, especially a semi-circular cross-section.

The case and the heat sink can have a square or rectangular cross-section.

The invention provides a method of preparing a shape memory alloy component for use in a surgical procedure, using a case and a heat sink which can be fitted snugly in the case which is formed from a metal, the heat sink having at least one recess cut into it in which the component can be fitted, which includes the steps of:
a. placing the component in heat exchange relationship with the heat sink,
b. placing the heat sink in the case,
c. manipulating the case.

Manipulating the case can involve placing the case and the heat sink in a controlled temperature storage unit (for example a freezer) after the component has been placed in the recess in the heat sink. For example, this can be used to reduce the temperature of the heat sink to a temperature which is less than the M_{f} temperature of the alloy.

The method can involve placing the component in the recess in the heat sink after the heat sink has been cooled, for example to reduce the temperature of the component to a level which is less than the M_{f} temperature of the alloy.

Accordingly, the invention provides a method of preparing a shape memory alloy component for use in a surgical procedure, using a case and a heat sink which can be fitted snugly in the case which is formed from a metal, the heat sink having at least one recess cut into it in which the component can be fitted, which includes the steps of:
a. cooling the heat sink while located within the case,
b. manipulating the case,
c. removing the heat sink from within the case
d. placing the component in heat exchange relationship with the heat sink.

Preferably, the case and the heat sink are provided in one or more sterile packages.

Embodiments of the invention will now be described by way of example with reference to the accompanying drawings, in which:
Figure 1 is a sectional elevation through a first embodiment of a case and heat sink for a system according to the present invention.
Figure 2 is an isometric view of a connector feature of the heat sink of the system shown in Figure 1.
Figure 3 is sectional elevation through a second embodiment of a case and heat sink for a system according to the present invention.

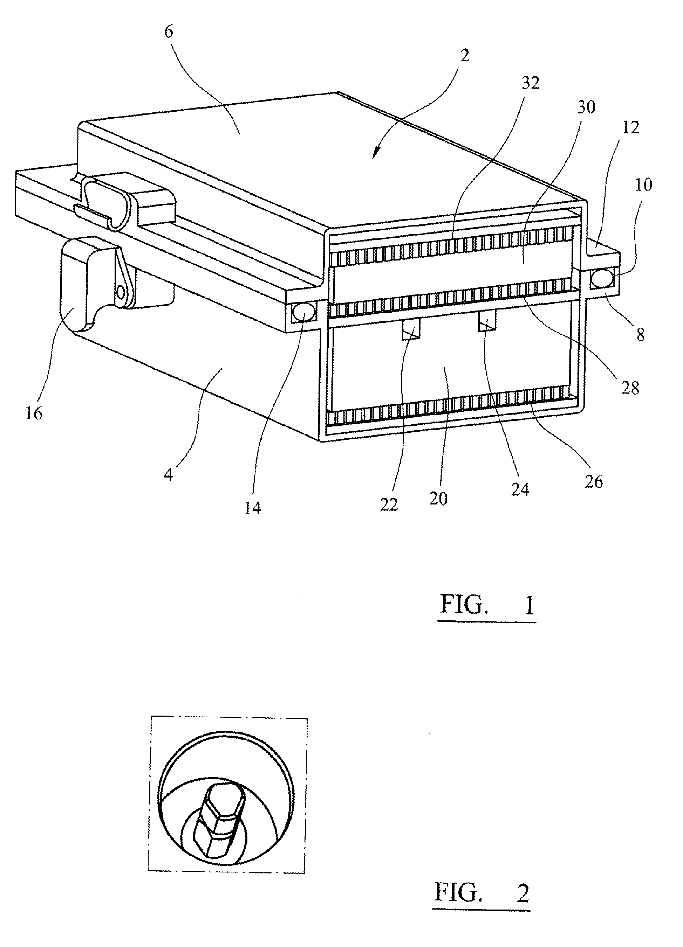

Referring to the drawings, Figure 1 shows a packaging system for a shape memory alloy rod which is for implantation in a patient in the treatment of a spinal deformity.

The system comprises a case 2 which comprises a base part 4 and a lid part 6. The base part has a peripheral flange 8 which has a groove 10 formed in it. The lid part has a mating flange 12 which can be aligned with the flange on the base part. An O-ring 14 formed from a silicone rubber is located in the groove 10. The diameter of the O-ring is slightly greater than the depth of the groove. The base and lid parts of the case 2 are formed from aluminium.

A series of latches 16 is provided spaced apart around the periphery of the case for clamping the flanges 8, 10 on the base and lid parts of the case together.

A base part 20 of a heat sink is provided within the base part 4 of the case. The base part of the heat sink is a block of aluminium which has a rectangular cross-section. The dimensions of the block are 350 mm × 25 mm × 95 mm. The block has two grooves 22, 24 machined into its top face, each of them being 7 mm wide and 7 mm deep, to accommodate respective rods (not shown) having lengths 300 mm and 150 mm respectively.

Layers 26, 28 of a silicone rubber separator are positioned on the floor of the base part of the case (between the case and the heat sink) and on the heat sink respectively. The separator material comprises a sheet with a plurality of studs moulded into it, so that it is resiliently deformable when subjected to a compressive force applied through its thickness.

A lid part 30 of a heat sink is provided within the lid part 6 of the case. The lid part of the heat sink is a block of aluminium which has a rectangular cross-section. The dimensions of the block are 350 mm × 15 mm × 95 mm. A layer 32 of the silicone rubber separator is provided between the base of the lid part of the case and the lid part of the heat sink.

Each of the base and lid parts of the heat sink has two quick connect studs (as shown in Figure 2) which can be engaged by an instrument using a standart AO type connection. This can enable quick connection between the instrument and the heat sink, protecting the user from exposure to low temperatures (after the heat sink has been cooled) and helping to maintain sterility.

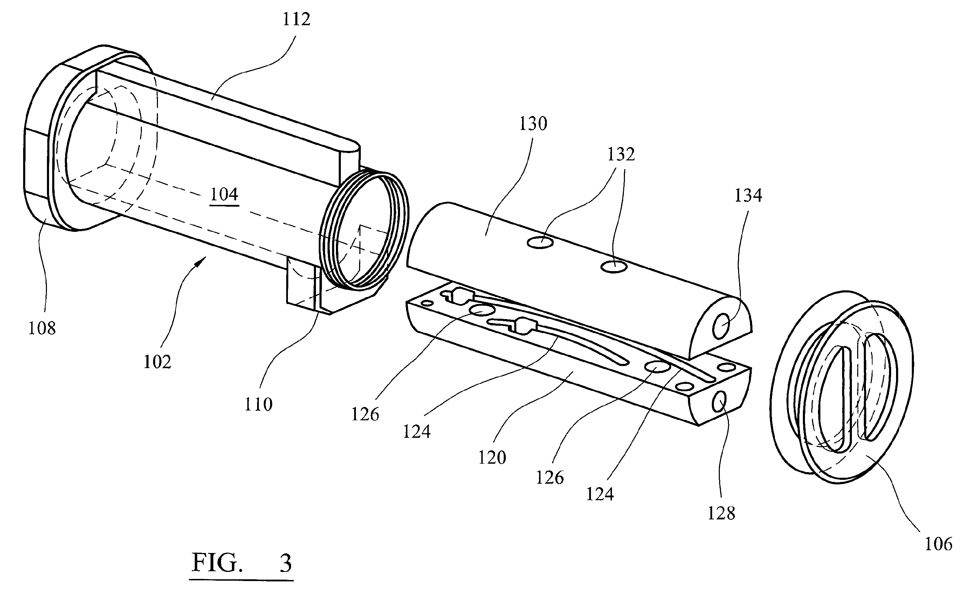

Figure 3 shows components of another embodiment of a packaging system according to the invention, in which the same reference numerals are used where possible to identify components which correspond to the components of the system shown in Figure 1.

The system comprises a case 102 which comprises a base part 104 and a lid part 106. The base part is tubular with a circular cross-section. It has two feet 108, 110 to enable it to be set down on a surface, and handle 112 for transporting it. The base and lid parts of the case 102 are formed from aluminium. The lid part engages the base part by means of cooperating threads.

The system includes a heat sink which comprises base and lid parts 120, 130. Each of the base and lid parts of the heat sink is formed from aluminium, as a half cylinder. Recesses 124 are provided in the base part 102 of the heat sink to accommodate rods which are formed from a shape memory alloy and other components.

The base part of the heat sink block has two quick connect studs 126 (as shown in Figure 2) in its upper face and a quick connect stud 128 in its end face which can be engaged using an instrument using a standard AO type connection.

The lid part of the heat sink has two quick connect studs 132 (as shown in Figure 2) in its upper face and a quick connect stud 134 in its end face which can be engaged using an instrument using a standard AO type connection.

Spacers can be provided between the heat sink and the case to reduce heat transfer between the case and the heat sink. Spacers can also help to reduce the likelihood of the heat sink becoming stuck to the case. Spacers can be made from a polymeric material, which can be provided locally on the inner wall of the case or the external surface of the heat sink, for example as studs or as strips. Examples of suitable materials include certain polyolefins (especially polyethylenes and polypropylenes), polyesters, polyamides, and polycarbonates. Particularly preferred materials include certain polyoxymethylenes in view of their low specific heat capacity, low thermal conductivity, light weight and ability to withstand sterilisation conditions of high temperature and pressure such as are encountered during sterilisation using an autoclave.

Use of the system of the invention can involve the following steps:
1. All parts if the system are put in standard instrument tray (that is subsequently covered with sterilization wrapping) that is then sterilized by exposure to heat and pressure.
2. At the day of the surgery, the sterilized wrapped instrument tray is opened and the parts of the system made available to the scrub nurse in the sterile field.
3. The scrub nurse assembles the parts of the system together; ensuring the sterile outer barrier provided by the case is then closed around the inner liner parts to protect their sterility.
4. The scrub nurse transfers the closed system to the running nurse.
5. The running nurse takes the close system and places it in a -25°C freezer for storage of a minimum of 30 minutes to cool the liner parts to -25°C.
6. The running nurse brings back the -25°C closed system into the operating theatre on the edge of the sterile field, opens the sterile outer barrier provided by the case so that the scrub nurse can retrieve the chilled sterile liner parts (the scrub nurse will use quick connect handles to carry the chilled liner parts so to prevent cool burning of her hands).
7. The scrub nurse places the liner base on the table, places the rod inside the groove in the base, place the liner lid on the base, waits for 5 minutes, opens the lid, and then retrieve the rod that is now cool and ready for shaping by the surgeon.

## Claims

1. A packaging system for shape memory alloy components for use in a surgical procedure, which comprises:
a. a case comprising a base part and a lid part and a seal to prevent ingress of contaminants when the base and lid parts are closed,
b. a heat sink which can be fitted snugly in the case and which is formed from a metal, and
c. at least one component which is formed from a shape memory alloy, which can be fitted in the case in heat exchange relationship with the heat sink.

2. A packaging system as claimed in claim 1, in which the heat sink has a recess formed in it, and in which the shape memory alloy component can be fitted in the recess.

3. A packaging system as claimed in claim 2, in which the component is in the form of a rod and the recess in the heat sink is in the form of a groove.

4. A packaging system as claimed in claim 3, in which the recess includes an enlarged portion in a local region along the length of the groove, to facilitate gripping of a rod which is located in the groove.

5. A packaging system as claimed in claim 1, in which the heat sink comprises a base part which can be fitted into the base part of the case and a lid part which can be fitted into the lid part of the case.

6. A packaging system as claimed in claim 5, in which the base and lid parts of the heat sink are formed from a metal.

7. A packaging system as claimed in claim 1, in which the metal of the heat sink has a specific heat capacity of at least about 400 J.kg⁻¹.K⁻¹.

8. A packaging system as claimed in claim 1, in which the metal of the heat sink has a thermal conductivity of at least about 15 W.m⁻¹.K⁻¹.

9. A packaging system as claimed in claim 1, in which the ratio of the volume of the recess in the heat sink to the volume of the heat sink is not more than about 0.1.

10. A packaging system as claimed in claim 1, in which the heat sink has an instrument recess formed in it, and in which the packaging system includes an instrument which can be positioned in the recess to engage the heat sink, so that the heat sink can be manipulated.

11. A packaging system as claimed in claim 1, in which at least one of the base part and the lid part of the case is formed from a metal.

12. A packaging system as claimed in claim 1, which includes a separator provided by a layer of an insulating material which can be positioned between the heat sink and the case.

13. A packaging system as claimed in claim 12, in which the insulating material comprises a silicone rubber.

14. A packaging system as claimed in claim 1, in which the rod and the groove have approximately constant cross-sections along most of their lengths, and in which the ratio of the cross-sectional area of the rod to the ratio of the cross-sectional area of the groove is at least about 0.6.

15. A packaging system as claimed in claim 1, in which the heat sink has at least two grooves formed in it, and in which the packaging system includes two rods which are formed from a shape memory alloy, which can be fitted in respective ones of the grooves in the heat sink.
